(19) Europäisches Patentamt
European Patent Office
Office européen des brevets

(11) **EP 2 399 601 A1**

(12) **EUROPEAN PATENT APPLICATION**

(43) Date of publication:
**28.12.2011 Bulletin 2011/52**

(21) Application number: **10006608.3**

(22) Date of filing: **24.06.2010**

(51) Int Cl.:
*A61K 38/48* (2006.01)  *A61P 43/00* (2006.01)
*A61P 21/02* (2006.01)  *A61P 17/00* (2006.01)
*A61Q 19/08* (2006.01)

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicant: **Merz Pharma GmbH & Co. KGaA 60318 Frankfurt (DE)**

(72) Inventors:
• **Wilbert, Eugen, Dr.**
**665779 Kelkheim (DE)**

• **Benecke, Reiner, Prof.Dr.**
**18184 Neu Thulendorf (DE)**

(74) Representative: **Ricker, Mathias**
**Wallinger Ricker Schlotter Tostmann Patent- und Rechtsanwälte Zweibrückenstrasse 5-7 80331 München (DE)**

Remarks:
Amended claims in accordance with Rule 137(2) EPC.

(54) **Botulinum toxin therapy**

(57)    A method for treating a disease or condition caused by or associated with hyperactivity of muscles or exocrine glands in a patient, the method comprising administering a composition comprising an effective amount of *Clostridium botulinum* toxin, wherein the composition is administered in such a way to the treated hyperactive cholinergic innervated muscle or gland that the of symptoms is kept stable during the whole treatment period.

EP 2 399 601 A1

## Description

### FIELD OF THE INVENTION

The present invention relates to methods for treating diseases or conditions by administering a composition containing the neurotoxic component of a *Clostridium botulinum* toxin complex, wherein the composition is devoid of any other protein of the Clostridium botulinum toxin complex and wherein the composition is administered at comparably short intervals via a specific administration scheme.

In one embodiment the administration scheme is carried out by an automated device, e.g. an implanted automatic pump device.

### BACKGROUND OF THE INVENTION

Botulinum toxin is produced by the bacterium *Clostridium.* There are seven antigenically distinct serotypes of botulinum toxin, namely botulinum toxin A, B, C, D, E, F and G. Botulinum toxins, when released from lysed Clostridium cultures are generally associated with other bacterial proteins, which together form a toxin complex. The neurotoxic subunit of this complex is referred herein as the "neurotoxic component" of the botulinum toxin complex. The proteins of the toxin complex are not toxic themselves but provide stability to the neurotoxic component and are responsible for oral toxicity in botulinum intoxications. Unlike the toxin complex, the neurotoxic component in its isolated and pure form, i.e. devoid of any complexing clostridial proteins, is acid labile and does not resist the aggressive environment in the gastrointestinal tract. The production of Botulinum toxin is known in the art, e.g. from WO 2008/000490.

Despite its toxic effects, botulinum toxin complex has been used as a therapeutic agent in a large number of diseases. Botulinum toxin serotype A was approved for human use in the United States in 1989 for the treatment of strabism, blepharospasm, and other diseases. It is commercially available as Botulinum toxin A protein complex, for example, under the tradename BOTOX (Allergan Inc.) or under the tradename DYSPORT (Ipsen Ltd). A composition comprising the pure neurotoxic component of serotype A in a composition that is devoid of any other proteins of the *Clostridium botulinum* toxin complex is commercially available under the trade name XEOMIN from Merz Pharmaceuticals.

For therapeutic application the Botulinum toxin is injected directly into the muscle or gland to be treated. The effect of Botulinum toxin is only temporary, which is the reason why repeated administration of Botulinum toxin may be required to maintain a therapeutic effect.

In the prior art one approach of administration botulinum toxin in short invervals (less than 12 weeks) is known. WO 2008/000490 relates to methods for treating diseases and disorders by administering a composition containing the neurotoxic component of a Clostridium botulinum toxin complex, wherein the composition is devoid of any other protein of the Clostridium botulinum toxin complex and wherein the composition is administered at short intervals and/or in high doses.

### OBJECTS OF THE INVENTION

The present invention allows for the treatment of a disease or condition caused by or associated with hyperactive cholinergic innervation of muscles or exocrine glands in a patient, by administering a composition comprising an effective amount of *Clostridium botulinum* toxin, wherein the composition is administered in such a way to the treated hyperactive cholinergic innervated muscle or gland that a significant reduction up to the full elimination of symptoms may be achieved during the whole treatment period. In a more general way, the invention aims to provide the patient with a more tailor-made administration of the neurotoxic component in order to improve the quality of life over the whole treatment period compared to the "classical" administration of the toxin once every three months. The present invention is based on the finding that the repeated administration of botulinum toxin in rather short intervals up to continuous administration is to be preferred, because it does not only lead to a stable improvement of the treated disease, but in some cases may lead to a full remission of the disease or condition symptoms.

It is noteworthy that the concept of the present invention, which involves the administration of the neurotoxic component of the botulinum toxin complex, generally allows the treatment of any condition which is associated with hyperactive cholinergic innervation of a muscle or an exocrine gland, where the neurotoxic component blocks acetylcholine secretion into the synaptic cleft.

### SUMMARY OF THE INVENTION

In one embodiment, the invention relates to a method for treating a disease or condition caused by or associated with hyperactive cholinergic innervation of muscles or exocrine glands in a patient, the method comprising administering a neurotoxic component of a *Clostridium botulinum* toxin complex, the component being devoid of any other protein component of the *Clostridium botulinum* toxin complex, wherein the component is administered via a scheme comprising

(a) a first treatment period comprising one or more treatment session(s) wherein in said first treatment period a first amount [units] of said neurotoxin component is administered, said first amount being sufficient to significantly reduce the symptoms of the treated disease or condition;
(b) at least one second treatment period of 12 weeks commencing immediately after said first treatment period comprising at least two further treatment sessions wherein in said second treatment period a second amount [units] of said neurotoxic component is administered, wherein the second amount [units] administered during said second treatment period is within 50% to 200% of the first amount administered during the first treatment period.

In a further embodiment the invention relates to a method of removing facial lines or wrinkles of the skin or facial asymmetries caused by or associated with hyperactive cholinergic innervation of muscles or exocrine glands in a patient, the method comprising administering a neurotoxic component of a *Clostridium botulinum* toxin complex, the component being devoid of any other protein component of the *Clostridium botulinum* toxin complex, wherein the component is administered via a scheme comprising

(a) a first treatment period comprising one or more treatment session(s) wherein in said first treatment period a first amount [units] of said neurotoxin component is administered, said first amount being sufficient to significantly reduce the symptoms of the treated disease or condition;
(b) at least one second treatment period of 12 weeks commencing immediately after said first treatment period comprising at least two further treatment sessions wherein in said second treatment period a second amount [units] of said neurotoxic component is administered, wherein the second amount [units] administered during said second treatment period is within 50% to 200% of the first amount administered during the first treatment period.

In another embodiment during said second treatment period the further treatment sessions are performed in time intervals of 6 weeks or less.
In a still further embodiment during said second treatment period the further treatment sessions are performed in time intervals of 14 days or less.
In one embodiment said second treatment period is repeated over a period of at least 1 year.
In another embodiment 20 units or less are administered per treatment session.
In another embodiment said second amount [units] administered during said second treatment period is within 50% to 150% of the first amount administered during the first treatment period.
In a further embodiment the second amount is administered by an automated device.
In a still further embodiment the automated device is a micro pump-device.
In one embodiment the administration is continuous.
In another embodiment the administration is performed by repeated injection.
In a further embodiment the injection is chosen among intradermal, subcutaneous, and intramuscular injection.
In a still further embodiment the administration is intermittent.
In a still further embodiment the intermittent administration is a repeated administration in intervals of less than 72 hours.
In a further embodiment the hyperactive exocrine gland is an autonomic exocrine gland and wherein the composition is administered into or in the vicinity of that gland.
In further embodiment the gland is selected from the group consisting of sweat gland, tear gland, salivary gland and mucosal gland; or the gland is associated with a disease or condition selected from the group consisting of Frey syndrome, Crocodile Tears syndrome, axillar hyperhidrosis, palmar hyperhidrosis, plantar hyperhidrosis, hyperhidrosis of the head and neck, hyperhidrosis of the body, rhinorrhea, or relative hypersalivation in patients with stroke, Parkinson's disease or Amyotrophic Lateral Sclerosis.
In a still further embodiment the disease or condition is or involves dystonia of a muscle.
In a still further embodiment the dystonia is selected from the group consisting of

(1) cranial dystonia including blepharospasm, oromandibular dystonia of the jaw opening or jaw closing type, bruxism, Meige syndrome, lingual dystonia, apraxia of eyelid opening, (2) cervical dystonia including antecollis, retrocollis, laterocollis, torticollis, (3) pharyngeal dystonia, (4) laryngeal dystonia including spasmodic dysphonia of the adductor type or of the abductor type, spasmodic dyspnea, (5) limb dystonia including arm dystonia such as task specific dystonias, including writer's cramp, musician's cramps or golfer's cramp, leg dystonia involving thigh adduction, thigh abduction, knee flexion, knee extension, ankle flexion, ankle extension or equinovarus deformity, foot dystonia involving striatal toe, toe flexion or toe extension, axial dystonia such as Pisa syndrome or belly dancer dystonia, segmental dystonia, hemidystonia or generalised dystonia, (6) dystonia in Lubag, (7) dystonia in corticobasal degeneration (8) tardive dystonia, (9) dystonia in spinocerebellar ataxia, (10) dystonia in Parkinson's disease, (11) dystonia in Huntington's disease, (12) dystonia in Hallervorden Spatz disease, (13) dopa-induced dyskinesias/dopa-induced dystonia, (14) tardive dyskinesias/tardive dystonia, (15) paroxysmal dyskinesias/dystonias (kinesiogenic, non-kinesiogenic, action-induced); or involves a clinical pattern selected from the group consisting of torticollis, laterocollis, retrocollis, anterocollis, flexed elbow, pronated forearm, flexed wrist, thumb-in-palm or clenched fist.

In another embodiment the muscle is selected from the group consisting of ipsilateral splenius, contralateral sternocleidomastoid, ipsilateral sternocleidomastoid, splenius capitis, scalene complex, levator scapulae, postvertebralis, ipsilateral trapezius, levator scapulae, bilateral splenius capitis, upper trapezius, deep postvertebralis, bilateral sternocleidomastoid, scalene complex, submental complex, brachioradialis, biceps brachialis, pronator quadratus pronator teres, flexor carpi radialis, flexor carpi ulnaris, flexor pollicis longus, adductor pollicis, flexor pollicis brevis/opponens, flexor digitorum superficialis, flexor digitorum profundus.

In a further embodiment the disease or condition is or involves spasticity of a muscle.

In a still further embodiment the spasticity is or is associated with (1) a spastic condition in encephalitis and myelitis relating to (a) autoimmune processes including respect to multiple sclerosis, transverse myelitis, Devic syndrome, (b) viral infections, (c) bacterial infections, (d) parasitic infections or (e) fungal infections, (2) hereditary spastic paraparesis, (3) postapoplectic syndrome resulting from hemispheric infarction, brainstem infarction or, myelon infarction, (4) a central nervous system trauma involving e.g. a hemispheric lesion, a brainstem lesion, a myelon lesion, (5) a central nervous system hemorrhage such as an intracerebral hemorrhage, a subarachnoidal hemorrhage, a subdural hemorrhage or an intraspinal hemorrhage, or (6) a neoplasia, e.g. a hemispheric tumor, a brainstem tumors or a myelon tumor or (7) post-stroke spasticity, or (9) spasticity caused by cerebral palsy.

In a still further embodiment the muscle is a smooth or striated muscle.

In a further embodiment the invention relates to a method of removing facial lines or wrinkles of the skin or facial asymmetries caused by or associated with hyperactive cholinergic innervation of muscles or exocrine glands in a patient, the method comprising administering a composition comprising an effective amount of *Clostridium botulinum* toxin, wherein the composition is administered in such a way that the full elimination of facial lines or wrinkles is kept stable during the whole treatment period.

In a still further embodiment of said method of removing facial lines or wrinkles of the skin or facial asymmetries caused by or associated with hyperactive cholinergic innervation of muscles or exocrine glands in a patient the composition is administered into the frown line, horizontal forehead line, crow's feet, nose perioral fold, mental ceases, popply chin, and/or platysmal bands.

In a still further embodiment of said method of removing facial lines or wrinkles of the skin or facial asymmetries caused by or associated with hyperactive cholinergic innervation of muscles or exocrine glands in a patient the injected muscle is selected from the group consisting of corrugator supercillii, orbicularis oculi, procerus, venter frontalis of occipitofrontalis, orbital part of orbicularis oculi, nasalis, orbicularis oris, depressor angulis oris, mentalis and platysma.

In one embodiment the neurotoxic component of the Botulinum toxin complex free of any non-toxic protein of the Botulinum toxin complex is selected from the group consisting of type A, B, C, D, E, F, G or a mixture thereof.

In another embodiment the neurotoxic component of the Botulinum toxin complex free of any non-toxic protein of the Botulinum toxin complex is of type A.

In a still further embodiment the neurotoxic component is a functional fragment of the native neurotoxic component.

In a still further embodiment the neurotoxic component is chemically or biologically modified.

In a still further embodiment the patient is human.

## DETAILED DESCRIPTION OF THE INVENTION

The present invention envisages treating patients characterized by having a disease or condition associated with hyperactive cholinergic innervation of muscles or exocrine glands.

In particular, the present invention relates to a method of treating a disease or condition caused by or associated with hyperactive cholinergic innervation of muscles or exocrine glands in a patient, the method comprising administering a neurotoxic component of a *Clostridium botulinum* toxin complex, the component being devoid of any other protein component of the *Clostridium botulinum* toxin complex, wherein the component is administered via a scheme comprising

(a) a first treatment period comprising one or more treatment session(s) wherein a first amount [units] of said neurotoxin component is administered, said first amount being sufficient to significantly reduce the symptoms of the treated disease or condition;
(b) at least one second treatment period of 12 weeks commencing immediately after said first treatment period comprising at least two further treatment sessions, wherein a second amount [units] of said neurotoxic component is administered, and wherein the second amount [units] administered during said second treatment period is within 50% to 200 % of the first amount administered during the first treatment period;
and optionally

(c) one or more further second treatment period(s) similar to (b), i.e. of 12 weeks length and comprising at least two treatment sessions and the administration of a total amount of the neurotoxic component of within 50 to 200 % of the first amount administered during the first treatment period.

Within this document, the term "disease" is defined as an abnormal condition of an organism that impairs bodily functions, associated with specific symptoms and signs. It may be caused by external factors, such as invading organisms, or it may be caused by internal dysfunctions. The term "disorder" is used hereinunder in terms of a "medical disorder", therefore it is synonymously to the term "disease". The term "condition" on the other hand is defined as an undesired state of being, comprising also non-medical conditions, like for example wrinkles, facial lines and facial asymmetries.

The term "patient" as used herein refers to mammals, human or animal, who suffered from one or more diseases or conditions specified hereinunder. There exist patients who have never been exposed to botulinum toxin but also to patients who have been exposed to botulinum toxin. The latter patient may have developed antibodies directed against the botulinum toxin complex or its components. Such antibodies may be neutralizing antibodies. Preferably, the patients do not have an antibody titer above 7mU, in particular not a titer of neutralizing antibodies above 7mU. The term "antibody titer not above ..." means an antibody concentration of less than 7mU, e.g. 1 mU to 6mU or 0.01 mU to 1 mU.

The term "mammal" as used herein refers to a class of vertebrate animals whose name is derived from their distinctive feature, mammary glands, with which they feed their young. They are also characterized by the possession of sweat glands, hair, three middle ear bones used in hearing, and a neocortex region in the brain. The term "mammals" also encompasses humans.

The terms "neurotoxic component" or "neurotoxin" as used throughout the specification, relates to the subunit of the botulinum toxin complex which has a neurotoxic activity and which has a molecular weight of approximately 150kDa in serotype A. The term "neurotoxic component" also includes the functional homologs found in the other serotypes of *Clostridium botulinum.* As mentioned above, in one embodiment of the present invention, the neurotoxic component is devoid of any other C. *botulinum* protein, especially also devoid of RNA potentially associated with the neurotoxic component.

The biological activity is commonly expressed in Mouse Units (MU). As used herein, 1 MU is the amount of neurotoxic component, which kills 50% of a specified mouse population after intraperitoneal injection, i.e. the mouse i.p. $LD_{50}$ (Schantz & Kauter, 1978). The terms "MU" and "Unit" or "U" are interchangeable. Alternatively, the biological activity may be expressed in Lethal Dose Units (LDU)/ng of protein (i.e. neurotoxic component). The term "MU" is used herein interchangeably with the terms "U" or "LDU".

Also included are genetically modified neurotoxic components containing 1, 2, 3, 4, 5, 6, 7, 8, 9, 10 or up to 20 amino acid mutations. A mutation may be a substitution, an insertion, an addition or a deletion. The mutation does not compromise any of the biological activities indicated above. However, it is also envisaged to use mutations to modulate the biological activity of the neurotoxic component.

In addition thereto, modified as well as recombinant produced neurotoxic components of botulinum toxins including the respective mutations, deletions, etc. are also within the scope of the present invention. With respect to suitable mutants, reference is made to WO 2006/027207 A1, WO 2006/114308 A1 and WO 2009/015840 which are fully incorporated by reference herein. Furthermore, within the present invention, mixtures of various serotypes (in the form of the botulinum toxin, the neurotoxic component or recombinant form or e.g. mixtures of botulinum toxins of types A and B) may be used. The present invention, however, also refers to botulinum toxins which are chemically modified, e.g. by pegylation, glycosylation, sulfatation, phosphorylation or any other modification, in particular of one or more surface or solvent exposed amino acid(s).

In another embodiment the botulinum toxin is a polypeptide comprising: a heavy chain-domain or fragment thereof of the neurotoxic component of a clostridial toxin; and a first light chain domain or fragment thereof, and at least one further light chain domain or fragment thereof, wherein the first and second light chain domain may be the same or different from each other. Such a polypeptide is disclosed for example in WO 2010/022979 which is fully incorporated by reference herein.

Also included are botulinum toxins containing chemically modified amino acids, for example one or more amino acids which are glycosylated, acetylated or otherwise modified, which may be beneficial to the uptake or stability of the toxin. Particularly the lipidation of the neurotoxic component is envisaged. Modifying residues may be added to the botulinum toxin e.g. by means of an enzymatic *in vitro* reaction or by using appropriate chemical reaction conditions. Alternatively, modifying enzymatic functions may be provided *in trans* by expressing the enzyme within the host cell. In another embodiment the neurotoxic component is modified to reduce its immunogenicity. Such a polypeptide is disclosed for example in PCT/EP2009/006273 which is fully incorporated by reference herein.

The term "treatment period" is used in this application to emphasize one group of "treatment sessions". The first treatment period, as envisaged by this invention, aims for the significant reduction up to the full elimination of symptoms of the treated disease or condition, whereas the second (and further) treatment period(s) start(s) immediately after and aim(s) for constantly keeping the patient free of said symptoms i.e. to prevent the recurrence of said symptoms of the treated disease or condition.

The term "treatment session" as used in this application, refers to one administration session, wherein the desired amount of the neurotoxic component is administered. A single treatment session may involve one or more injection(s). As such the term "treatment session" describes a period in time during which the physician or an automated device administers one fraction of the desired total amount of the neurotoxic component. It is therefore different to a "treatment period", which describes the administration of the total (first, second or subsequent) amount of the neurotoxin, including first and subsequent treatment sessions and may last between a few days (in the case of the first treatment period) and up to 12 weeks (in case of the second treatment period).

A treatment session might comprise several injections of an amount of the neurotoxin to different parts of one muscle or to several muscles. Such a treatment is normally performed within a few minutes, but might in complicate cases take up to one hour. The individual length of a treatment session is therefore defined by the physician and the complexity of muscle hyperactivity pattern.

For example the treatment of M. sternocleidomastoideus, M. splenius capitis, M. semispinalis capitis and M. trapezius may involve 1, 2, 3, 4 or 5 injections in different localizations, whereas the treatment of M. levator scapulae or M. scaleni may involve only 1 to 3 injections. The amount used for treatment is dependent on a number of parameters, which are known in the art. Such parameters include for example the size of the muscle, the serotype of the neurotoxic component, the target tissue to be injected and a number of patient specific factors.

In such cases, wherein the neurotoxin is administered to prevent the recurrence of the symptoms of the treated disease or condition, one treatment session comprises the number of injections the physician needs to achieve the desired effect of preventing the recurrence of said symptoms.

For reasons of simplification the term "treatment session" is also used in conjunction with automated devices. There, however, one treatment session is defined hereinunder as "one treatment per hour". For example, if an automated device administers an amount once per day split in 24 single fractions applied within one hour, all 24 administrations together are considered "one treatment session". If, however, the automated device administers once per hour one fraction, resulting in 24 times per day, said pattern is considered as 24 "treatment sessions" per day. Furthermore, if the automated device administers a continuous flow of neurotoxin, then each hour would be seen as one "treatment session", resulting also in 24 treatment sessions per day.

As used throughout the specification of the present invention, the term "first amount" or "second amount" or "consecutive amount" refers to the total dosage administered during the respective treatment period and means the sum of neurotoxin applied to a patient during the respective treatment period. This "total amount" is normally split into fractions which are applied during each the treatment session.

In some embodiments automated devices, such as implanted pump-devices are used for administration. With such devices it is particularly possible to maintain the paralyzed state of the treated muscle or gland over a long period of time, i.e. 1 year, 2 years, 3 years and up to 5 years.

In one embodiment the first treatment period comprises one first treatment session where a first amount [units] of the neurotoxic component is administered, and wherein the first amount [units] is high enough to significantly reduce up to fully eliminate the symptoms of the treated disease or condition.

In another embodiment the first treatment period comprises more than one treatment sessions. Such an administration scheme is used to "converge" to the "optimal dose". For example, in a first treatment session a suboptimal dose of the neurotoxic component is administered. Should the patient's disease symptoms not sufficiently respond, more neurotoxic component is administered in a second or in subsequent treatment session(s). In doing so, the further dosages will be reduced step by step to that extend necessary to just significantly reduce the symptoms of the treated disease or condition. Moreover, based on this embodiment of the method it is possible to more effectively treat a patient in need of an additional administration of the neurotoxic component. This is the case, e.g. when, after a first or previous treatment, it is established that additional muscles contribute to the disease symptoms or when muscles have been missed. Since according to the present invention the total amount is split into a first treatment sessions and further "boost" sessions, large amounts, which would otherwise not be compliant when administered in a single treatment session, may be administered to a patient without observing significant adverse effects.

The term "suboptimal dose" as used herein refers to any dose at which not yet a full elimination of symptoms is reached, e.g. the spastic muscle still contracts, or wrinkles are still visible or dystonias are still present. Suboptimal dosages may be used by the skilled person to approach the optimal dose needed to fully eliminate the symptoms associated with a disease.

The term "optimal dose" refers to the dose where the symptoms of the disease/condition to be treated is significantly reduced, i.e. the symptoms are no longer visible and/or not recognizable by the patient. The term "optimal dose" also includes the dose where the symptoms are fully eliminated, i.e. are not detectable also by measurement by a physician.

In one embodiment the dosage needed to significantly reduce the symptoms of the treated disease or condition as well as the time-point at which an on-set of first symptoms is to be expected is determined by the physician.

Within the present invention, it is now possible to treat patients with far larger amounts of neurotoxic component. In adult patients, such amounts may for example exceed 500 U of neurotoxic component. It is also possible to treat the patients with a "tailor-made" dosage that may be chosen depending on e.g. patients constitution or severity of condition.

In one embodiment of the present invention said first amount which is administered exceeds 500 U in total in adults or 15 U/kg body weight in children.

In one embodiment of the present invention, the patient is a patient requiring high doses of neurotoxic component. In another embodiment of the present invention, (a) the patient is a patient with a severe movement disorder or severe spasticity and (b) the effective amount administered exceeds 500U of neurotoxic component in adults or exceeds 15 U/Kg body weight in children.

As used throughout the present invention, an amount exceeding 500 U is for example an amount of more than 500 U and up to 550 U, up to 600 U, up to 700 U, up to 800 U, up to 900 U, up to 1000 U, up to 1100 U, up to 1200 U, up to 1300 U, up to 1400 U, up to 1500 U, up to 1600 U, up to 1700 U, up to 1800 U, up to 1900 U, or up to 2000 U. In one embodiment the dose administered is in the range of 500 to 900 U, in another embodiment approximately 850 U. In children, "high amounts" means amounts exceeding 15 U/kg and up to 16 U/kg, up to 17 U/kg, up to 18 U/kg, up to 19 U/kg, up to 20 U/kg.

In addition to the above-referenced high dosing, it is also within the ambit of the present invention to provide a significantly less amount of the neurotoxic component as the first amount. This applies in particular with regard to the cosmetic treatments and the treatment of small muscles in general. Examples of said first amounts are 10 U, 20 U, 30 U, 50 U, 75 U, 100 U, 200 U and 300 U, or stated more generally 5 U to less than 500 U.

The second treatment period starts as soon as the significant reduction of the symptoms of the treated disease or condition are reached during the first treatment session (i.e. "commencing immediately after") and lasts for 12 weeks. The general concept of the second treatment period is to keep the muscle or gland in a steady-state of paralysis i.e. not to allow the symptoms of the treated disease or condition to recover. By this measure the involved muscles are kept in a reduced activity state, which leads to a significant reduction up to full elimination of the symptoms of the disease / condition treated. Any decrease of the therapeutic effect can be monitored by treatment calendars in which the patient records the severity of this disorder on a day-to-day basis.

During the second treatment period at least two treatment sessions are performed, comprising administering a second amount [units] of said neurotoxic component, wherein the total amount [units] administered during said second treatment period is within 50% to 200% of the first amount.

For example, if the first amount administered during the first treatment period comprises 100 units of the neurotoxic component, the second amount comprises between 50 and 200 units and is split in at least two treatment sessions within 12 weeks, e.g. in a first treatment session wherein 30 units are administered, and a second treatment session, wherein 20 units are administered.

In other embodiments the application of a few units in short intervals during the second treatment period is envisaged. In such embodiments the number of treatment sessions is higher than just 2 times in 12 weeks, for example 3, 4, 5, 6, 7, 8, 9, 10, 11, 12 times in 12 weeks; or 1, 2, 3, 4, 5, 6, 7 times per week; or 1, 2, 3, 4, 5, 6, 7, 8, 9, 10, 11, 12, 13, 14, 15, 16, 17, 18, 19, 20, 21, 22, 23, 24 times per day. In another embodiment even a continuous administration (via infusion or an automated device) is envisaged.

The term *"continuous administration"* as used herein means 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 times per hour. In all of these embodiments, however, the total amount of neurotoxic component during said second treatment period still is within 50% to 200% of the first amount. Therefore, if the amount of the first treatment period was 100 units, it is within the scope of this invention to administer e.g. every 6 hours, a small amount of e.g. 0.3 units to the site of treatment. In further embodiments amounts of 20 units or less, of 10 units or less, 5 units or less, 3 units or less, of 1 unit or less but more than at least 0,001 units are envisaged per treatment session.

In another embodiment an automated device such as a pump- or infusion-device is implanted which administers the desired amount of botulinum toxin units in the desired intervals or continuously. Such a device facilitates an automated repeated injection or continuous infusion therapy, respectively. This renders repeated injections performed by a physician unnecessary and greatly increases the compliance of the patient. In one embodiment such devices are used, if frequent administrations of small amounts [units] are desired.

Such a continuous administration might reduce the "toxic burden" onto the non-target tissue and immune system (e.g. because of diffusion effects etc.) even further. In such cases during the second treatment period a continuous flow of the botulinum toxin to the site of treatment is envisaged (e.g. via pump-devices and micro-catheters).

In some embodiments also the intermittent (i.e. periodical) and continuous administration, as described above, might be used alternating. This for example might be the case, if a pump device applies e.g. a daily dosage of botulinum toxin with a continuous flow of one hour per day. Also more complex administration patterns, for example train pulses, wherein a set of several administrations with small intervals is separated by larger intervals, are encompassed by this invention..

In one embodiment such an automated device is an implantable computerized drug-delivery system, micro-pump device or micro-infusion device, which allows the controlled release of the neurotoxin over time (cf. for example US 5860957, US 6017318). The administration of the botulinum toxin or botulinum toxin formulation might be also facilitated by a convection-enhanced delivery device (CED) (cf. e.g. WO 2009/023601). Although implanted devices are preferred, of course also external pump devices are encompassed by this invention.

In one embodiment also a feedback device is envisaged, comprising a sensor able to *in vivo* stimulate a muscle or gland and measure its reaction, a micro-pump device and a microprocessor which coordinates the release of the botulinum toxin upon the sensor input. If, for example, such a device would be used to treat a spastic muscle, it would preiodically stimulate the spastic muscle with small electric impulses and measure the muscle's reaction. At a certain threshold of muscle response the microprocessor would then initiate a release of small amounts of botulinum toxin to the site of treatment via the micro-pump device until the sensor does not recognize any reaction above the threshold level.

In another embodiment also retarded release devices or formulations such as polymer drug release and patches that slowly pass drugs into the body through the skin are envisaged and encompassed by this invention.

Furthermore, in one embodiment the administration of the botulinum toxin or botulinum toxin formulation is performed via a micro-catheter-like device (e.g. DE10/2006/020402, US2007/0265551), enabling either the skilled person or any automated device to reapply the botulinum toxin if needed.

The before mentioned automated delivery devices are preferably used for the "long-term application" of the botulinum toxin. The term "long-term application" herein refers to treatment periods of 1, 2, 3, 4, 5, 6, 7, 8, 9, 10, 11, 12 months up to 1, 2, 3, 4, 5, 6, 7, 8, 9, 10 years. In some embodiments, if a longer treatment than just 12 weeks is desired, further treatment periods might follow immediately after the second treatment period. In these cases the scheme of the second treatment period of 12 weeks is repeated, resulting in a total treatment of 6 months, 1 year, 2 years, 3 years and up to 10 years, 15 years, 20 years. Also during the consecutive treatment period(s) the consecutive amount(s) of neurotoxin administered in each treatment period is within $\pm$ 50% of the first amount of the first treatment period.

In all before mentioned cases, however, the second (subsequent) treatment is carried out at a point in time <u>before</u> the efficacy of the first (previous) treatment would begin to decline in the patient if the subsequent treatment would not be performed. This represents a significant difference towards the treatments known from the prior art, wherein the second (subsequent) treatment (S) have started after the efficacy of the first treatment already started to decline, i.e. after the so-called "Plateau-Effect" attributed to an action of Botulinum toxin sufficient to significantly reduce, up to fully eliminate, the symptoms of the disease already started to diminish. Such a time point could be derived from previous conventional treatments after which the patient started developing new symptoms at a certain time point. Also *in vivo* muscle or gland activity measurements may aid in finding the ideal time point of re-injection. In some cases even a full remission may be achieved when high frequency therapies lead to a constant plateau effect after such treatment period. In at least one case a full or complete remission was reached, i.e. the patient stayed free of symptoms even after the therapy with botulinum toxin was stopped for at least 6 months, preferably at least 1 year, up to life long. This finding is surprising and has not been reported in the prior art before.

With the treatment regimen of the present invention, a stable quality of life for the patients is achieved.

The determination of the parameter "stable quality of life" for the patients is exemplarily described hereinunder by reference to one condition to be treated according to the present invention, namely blepharospasm, on the basis of the so-called Blepharospasm Disability Index (BSDI). The Blepharospasm Disability Index [BSDI] is a self-rating scale for assessment of impairment of specific activities of daily living caused by BEB Goertelmeyer R, Brinkmann S, Comes G, Delcker A, The Blepharospasm Disability Index (BSDI) for the Assessment of Functional Health in Focal Dystonia, Clin. Neurophysiol. 2002; 113(1): S77-S78.

The scale is to be answered by the patient at each visit. It includes 6 items to be assessed with a 5-point listing (i.e., 0-4 points per item) ranging from "no impairment, "slight/moderate/severe impairment" and "no longer possible due to my illness". The 6 items are "Driving a vehicle", "Reading", "Watching TV", "Shopping", "Walking" and "Doing everyday activities". Unlike other functional scales, which ignore scaling in case of non-applicable items, the BSDI allows for answering as 'not applicable' for five items except "Doing everyday activities"

The BSDI mean score for non-missing items is calculated by adding all applicable and answered items, and dividing by the number of items answered.

However, such parameters are available for many other diseases and conditions to be treated within the present invention, e. g. craniocervical dystonia questionnaire (CCDQ 24) for cervical dystonia (Mueller J, Wissel J, Kemmler G, Bodner T, Poewe W, Quality of life in patients with craniocervical dystonia: development of the CCDQ-24, Mov. Disord. 2000; 15(Suppl 3): 761, and HRQL, by the Swedish Short Form 36 Health Survey Questionnaire (SF-36) for spasticity (Welmer AK, von Arbin M, Widen Holmqvist L, Sommerfeld DK, Spasticity and its association with functioning and health-related quality of life 18 months after stroke, Cerebrovasc. Dis. 2006; 21(4): 247-253).

At each of the re-injection treatment or last treatment of the patient, the difference $\Delta_{BSDI}$ between the actual BSDI observation and the BSDI value at baseline will be calculated:

$$\Delta_{BSDI} = BSDI_{actual} - BSDI_{baseline}$$

The $BSDI_{baseline}$ is determined during the first visit of the patient to be treated and before the first injection of the medicament. The $BSDI_{actual}$ is determined after the respective (re)-injection of the medicament and 3 weeks thereafter, respectively.

On the basis of the $\Delta_{BSDI}$ value recorded at the first treatment, each patient will be allocated to one of three strata in the following way:

- Stratum 1 (moderate improvement): $-1.00 \leq \Delta_{BSDI} \leq -0.65$
- Stratum 2 (marked improvement): $-1.35 \leq \Delta_{BSDI} < -1.00$
- Stratum 3 (abolishment of signs and symptoms): $\Delta_{BSDI} < -1.35$

A patient is classified as a responder, i.e., the patient demonstrates a stable level of quality of life, if none of the $\Delta_{BSDI}$ values calculated exceeds a threshold $\Delta_c$. The value of the threshold $\Delta_c$ depends on the stratum the patient belongs to. The values of the thresholds are:

- Stratum 1: $\Delta_c = -0.40$
- Stratum 2: $\Delta_c = -0.75$
- Stratum 3: $\Delta_c = -1.10$

Responders show a reduction of their baseline BSDI value, and therefore an improvement of their quality of life status. The minimal magnitude of the improvement is given by the threshold $\Delta_c$. The values of $\Delta_c$ decrease with the number of the stratum because patients in Stratum 2 show a stronger response to the initial injection than patients in Stratum 1 (resulting in lower BSDI differences), and patients in Stratum 3 react even stronger than patients in Stratum 2.

Finally, the observed differences between the BSDI level at the day of a re-injection treatment and the baseline BSDI level will be analyzed to investigate if there is any improvement over time of the quality of life level at the time of re-injection (expected time of waning of treatment effect).

The above specified (and claimed) modes of administration of the medicament as used within the present invention belong usually to the activities of the physicians treating patients. However, the mode of administration can be also part of the manufacture of the medicament in that e.g. the package of the medicament contains a specifically adapted leaflet with instructions to the physician and/or the patient and/or the package is specifically adapted to allow the mode of administration according to the present invention.

The inventive mode of administration, as adapted in a leaflet with instructions to the physician and/or the patient and/or the package, comprises a first treatment period comprising one or more treatment session(s), wherein a first amount [units] of said neurotoxin component is administered, said first amount being sufficient to fully eliminate the symptoms of the treated disease or condition; at least one second treatment period of 12 weeks commencing immediately after said first treatment period comprising at least two further treatment sessions wherein a second amount [units] of said neurotoxic component is administered, wherein the second amount [units] administered during said second treatment period is within $\pm$ 50% of the first amount administered during the first treatment period; and optionally one or more further second treatment period(s) similar to (b), i.e. of 12 weeks length and comprising at least two treatment sessions and the administration of a total amount of the neurotoxic component of within 50% to 200% of the first amount administered during the first treatment period.

The treatment offered by the present invention may be directed at any of the known indications, as e.g. described in detail in Dressler D (2000) (Botulinum Toxin Therapy. Thieme Verlag, Stuttgart, New York).

Particularly to be mentioned are dystonias, such as blepharospasm and cervical distonia; treatments of spastic muscles, such as juvenile cerebral palsy and post-stroke spasticity; the treatment of bladder disfunctions, such as detrusor sphincter dyssynergia and bladder sphincter spasm; prostate hyperplasia; treatment of vocal cords, such as abductor vocal cord paralysis. In addition, within the present invention a cosmetic use according to the administration skim of the invention is envisaged, such as the treatment of croaw's feet, frowning, facial asymmetries, mentalis dimples, frontal lines, masseter lift, platysma, smoker's lines and marionette lines.

This method of the present invention allows treating facial muscles or wrinkles of a patient's skin or a facial asymmetry. Typically, smaller amounts of neurotoxic component are used in such cosmetic treatment. Such amounts are preferably in the range of 1 to 5, 5 to 10, 10 to 20 or 20 to 50 Units. Such total amounts may be administered on the same day or on a subsequent day of treatment. For example, during a first treatment session a first fraction of the dose may be administered. This first fraction is preferably a suboptimal fraction, i.e. a fraction, which does not remove the wrinkles or skin lines completely. During one or more treatment sessions, the remaining fraction of the total dose may be administered.

The botulinum toxin referred to herein above, may be part of a composition or pharmaceutical composition. This pharmaceutical composition may contain additional pharmaceutically active components. "Pharmaceutical composition" is a formulation in which an active ingredient for use as a medicament or diagnostic is contained or comprised. Such pharmaceutical composition may be suitable for diagnostic or therapeutic administration (i.e. by intramuscular or subcutaneous injection) to a human patient. The pharmaceutical composition may be lyophilized or vacuum dried, reconstituted, or in solution. When reconstituted it is preferred that the reconstituted solution is prepared adding sterile physiological saline (0.9% NaCl). For further details regarding suitable composition reference is made to WO 2008/000490, the content of which is fully incorporated herein by reference.

In even another embodiment of the present invention, the pharmaceutical composition may comprise the neurotoxic component and a hyaluronic acid or a polyvinylpyrrolidone, such composition being optionally pH stabilized by a suitable pH buffer, in particular by a sodium acetate buffer, and / or a cryoprotectant polyalcohol.

The pharmaceutical composition of the present invention may comprise a botulinum toxin, and a hyaluronic acid. The hyaluronic acid stabilizes the botulinum toxin. The pharmaceutical compositions disclosed herein may have a pH of between about 4 and 7.5 when reconstituted or upon injection. The hyaluronic acid in the instant pharmaceutical composition is in one embodiment combined with the instant neurotoxic component in a quantity of 0.1 to 10 mg, especially 1 mg hyaluronic acid per ml in a 200 U/ml botulinum toxin solution. In another embodiment the subject solution also contains a 1-100 mM, especially 10 mM sodium acetate buffer.

The polyvinylpyrrolidone in the instant pharmaceutical composition is in one embodiment combined with the instant neurotoxic component in a quantity of 10 to 500 mg, especially 100 mg polyvinylpyrrolidone per ml in a 200 U/ml botulinum toxin solution. In another embodiment, the subject solution also contains a 1-100 mM, especially 10 mM sodium acetate buffer.

In one embodiment the formulation comprises a hydrophilic polymer, a mixture of a polyalcohol and a sugar, wherein the weight ratio of polyalcohol to sugar is from 2:1 to 5:1 (wt-%), a detergent, wherein said formulation is free of stabilising proteins. The hydrophilic polymer within said formulation may be selected from e.g. hyaluronic acid and/or polyvinylpyrrolidone.

## FURTHER DEFINITIONS

The term "about" as used in the context of the present invention means "approximately" or "nearly". In the context of numerical values, without committing to a strict numerical definition, the term may be construed to estimate a value that is +/-10% of the value or range indicated.

The term "hyperactive cholinergic innervation", as used herein, relates to a synapse, which is characterized by an unusually high amount of acetylcholine release into the synaptic cleft. "Unusually high" relates to an increase of up to 25%, up to 50% or more with respect to a reference activity which may be obtained, for example, by comparing the release with the release at a synapse of the same type but which is not in a hyperactive state, wherein muscle dystonia may be indicative of the hyperactive state. "Up to 25%" means, for example, about 1% to about 25%. Methods for performing the required measurements are known in the art.

The term "effective amount" means an amount of neurotoxic component, which, after administration, results in a partial or complete removal of disease symptoms. Effective amounts are generally in the range of 1 to 2000MU but also doses of up to 5000MU may be used.

In one embodiment the "effective amount" may be measured in vivo with muscle contraction assays, as for example disclosed in EP 2015065 or Sheridan et al., J. Appl. Toxicol., 19, S29-S33 (1999). In these publications two electrodes are placed near to the muscle, which is to be injected botulinum toxin, and its subsequent paralyzation is measured, allowing to find the dosage when the tonus of the muscle is semi- or fully paralyzed.

The term "muscle(s)" as used herein encompasses the contractile tissue of the body and is derived from the mesodermal layer of embryonic germ cells. Muscle cells contain contractile filaments that move past each other and change the size of the cell. They are classified as skeletal, cardiac, or smooth muscles. "Striated muscle", again, is a form of muscle fibers that are combined into parallel fibers (comprising skeletal and cardiac muscle).

The term "exocrine gland(s)" as used herein encompasses glands that secrete their products (hormones) into ducts (duct glands). They are the counterparts to endocrine glands, which secrete their products (hormones) directly into the bloodstream (ductless glands) or release hormones (paracrines) that affect only target cells nearby the release site.

The term "dystonia(s)" as used herein refers to a neurological movement disorder in which sustained muscle contractions cause twisting and repetitive movements or abnormal postures. The disorder may be inherited or caused by other factors such as birth-related or other physical trauma, infection, poisoning (e.g. lead poisoning) or reaction to drugs, particularly neuroleptics.

The term "spasticity(ies)" (or muscular hypertonicity) as used herein refers to a disorder of the central nervous system (CNS) in which certain muscles continually receive a message to tighten and contract. The nerves leading to those muscles, unable to regulate themselves (which would provide for normal muscle tone), permanently and continually "over-fire" these commands to tighten and contract. This causes stiffness or tightness of the muscles and interferes with gait and movement, and sometimes speech.

The term "wrinkle" as used herein refers to a fold, ridge or crease in the skin of the body.

The term "administering" or "administration" as used herein encompasses any method of bringing the botulinum toxin and its pharmaceutical compositions in contact with the site of treatment of the patient's body. In most cases such an administration will be an injection (e.g. intradermal, subcutaneous, intramuscular, intraosseous or intraperitoneal). However, also other routes of administration like e.g. transdermal, infusion, perfusion or transmucosal are encompassed by this invention.

The term "site of treatment" as used herein encompasses any muscle or gland as well as neighboring tissue which is treated to remedy the related disease.

The term "fully eliminate the symptoms" as used herein refers to a state of the muscle or gland associated with the treated disease or condition, where none of the symptoms normally associated with said disease or disorder can be recognized by the skilled person e.g. if a spastic muscle is treated no spastic can be recognized anymore or e.g. if a secreting significantly reduced gland is treated no secretion can be recognized anymore.

The term "intermittent" administration as used herein refers to an administration wherein between each treatment session an interval of time without administration occurs. The opposite is a "continuous" administration, wherein a steady flow of toxin to the site of treatment (measured in units per minute) is intended.

The term "vicinity" in terms of "vicinity to a gland" or "vicinity to a muscle" as used herein refers to any administration, wherein the botulinum toxin is applied not into said gland or muscle but in near neighborhood. Thereby the distance is just small enough, that the diffusion or other transport mechanisms in the body allow the botulinum toxin to affect the targeted muscle or gland. For example an injection into the vicinity of a gland might be preferred in cases where a direct injection into the gland would lead to tissue damages.

The term "functional fragment" refers to a fragment of the neurotoxic component of the botulinum toxin complex, which still possesses the proteolytic activity of the native neurotoxin. In one embodiment such a fragment is the light chain of the neurotoxic component, in other embodiments it is a fragment of the light chain, at least comprising the amino acids mediating the proteolytic activity of the native neurotoxin. In yet other embodiments, the fragment might also comprise domains of the heavy chain of the neurotoxic component. Functional fragments might be produced recombinantly, chemically or by enzymatic reactions.

## EXAMPLES

### Example 1

A 43-years-old patient developed within three weeks a typical sign of a torticollis spasmodicus with an inflection of the head to the right. On a severity scale of 0 to 15 the patient showed the value of 12 (Tsui scale). The patient's family history was negative for dystonic disorders. 14 days after onset of the symptoms a treatment with Xeomin of 200 U was performed for the first time. This led to an improvement of a time period of six to eight weeks each, afterwards the cervical dystonia returned quite rapidly to full extent. Because of the intermitting clear recurrence of the botulinum toxin effect the patient asked to reduce the time interval between each therapy from three to four months to four to six weeks. The high frequent botulinum toxin therapy was then performed for two years and led to development of a stable improvement of the torticollis spasmodicus with a turn of the head to the right. At the end of this two-years high frequency therapy the patient showed a picture of a full remission, making further treatments for more than one and a half year unnecessary because of the stable situation of the patient.

During the high frequent treatment the re-injection was performed in short intervals and each time with 100-150 U Xeomin.

**Claims**

1. A neurotoxic component of a *Clostridium botulinum* toxin complex, the component being devoid of any other protein component of the *Clostridium botulinum* toxin complex for the treatment of a disease or condition caused by or associated with hyperactive cholinergic innervation of muscles or exocrine glands in a patient, wherein the component is administered via a scheme comprising

 a first treatment period comprising one or more treatment session(s) wherein in said first treatment period a first amount [units] of said neurotoxic component is administered, said first amount being sufficient to significantly reduce the symptoms of the treated disease or condition;

 at least one second treatment period of 12 weeks commencing immediately after said first treatment period comprising at least two further treatment sessions wherein in said second treatment period a second amount [units] of said neurotoxic component is administered, wherein the second amount [units] administered during said second treatment period is within 50% to 200% of the first amount administered during the first treatment period.

2. The use of a neurotoxic component of a *Clostridium botulinum* toxin complex, the component being devoid of any other protein component of the *Clostridium botulinum* toxin complex for manufacturing a medicament for the treatment of a disease or condition caused by or associated with hyperactive cholinergic innervation of muscles or exocrine glands in a patient, wherein the component is administered via a scheme comprising

 a first treatment period comprising one or more treatment session(s) wherein in said first treatment period a first amount [units] of said neurotoxic component is administered, said first amount being sufficient to significantly reduce the symptoms of the treated disease or condition;

 at least one second treatment period of 12 weeks commencing immediately after said first treatment period comprising at least two further treatment sessions wherein in said second treatment period a second amount [units] of said neurotoxic component is administered, wherein the second amount [units] administered during said second treatment period is within 50% to 200% of the first amount administered during the first treatment period.

3. The neurotoxic component/use of claim 1 or 2, wherein during said second treatment period the further treatment sessions injections are performed in time intervals of 6 weeks or less.

4. The neurotoxic component/use of claim 1 or 2, wherein during said second treatment period the further treatment sessions are performed in time intervals of 14 days or less.

5. The neurotoxic component/use of any of the preceding claims, wherein said second treatment period is repeated over a period of at least 1 year.

6. The neurotoxic component/use of any of the preceding claims, wherein 20 units or less are administered per treatment session.

7. The neurotoxic component/use of any of the preceding claims, wherein said second amount [units] administered during said second treatment period is within 50% to 150% of the first amount administered during the first treatment period.

8. The neurotoxic component/use of any of the preceding claims, wherein said second amount is administered by an automated device.

9. The neurotoxic component/use of claim 8, wherein the automated device is a micro pump-device.

10. The neurotoxic component/use of claim 8 or 9, wherein the administration is continuous.

11. The neurotoxic component/use of any of the precedings claims, wherein the administration is performed by repeated injection.

12. The neurotoxic component/use of claim 11, wherein the injection is selected from the group comprising intradermal, subcutaneous, intramuscular and injection.

13. The neurotoxic component/use of any of the claims 1 to 9, 11 and 12, wherein the administration is intermittent.

14. The neurotoxic component/use of claim 13, wherein the intermittent administration is a repeated administration in intervals of less than 72 hours.

15. The use of a neurotoxic component of a *Clostridium botulinum* toxin complex, the component being devoid of any other protein component of the *Clostridium botulinum* toxin complex for the removal of facial lines or wrinkles of the skin or facial asymmetries, wherein the component is administered according to the embodiments defined in any of the preceding claims.


**Amended claims in accordance with Rule 137(2) EPC.**

1. A neurotoxic component of a *Clostridium botulinum* toxin complex, the component being devoid of any other protein component of the *Clostridium botulinum* toxin complex for the use in treating a disease or condition caused by or associated with hyperactive cholinergic innervation of muscles or exocrine glands in a patient, wherein the component is administered via a scheme comprising

a) a first treatment period lasting up to one hour comprising one or more treatment session(s) wherein in said first treatment period a first amount [units] of said neurotoxic component is administered, said first amount being sufficient to significantly reduce the symptoms of the treated disease or condition such that the symptoms are no longer visible and/or recognizable by the patient;
b) at least one second treatment period of 12 weeks commencing immediately after said first treatment period comprising at least two further treatment sessions wherein in said second treatment period a second amount [units] of said neurotoxic component is administered, wherein the second amount [units] administered during said second treatment period is within 50% to 200% of the first amount administered during the first treatment period.

2. The use of claim 1, wherein during said second treatment period the further treatment sessions injections are performed in time intervals of 6 weeks or less.

3. The use of claim 1, wherein during said second treatment period the further treatment sessions are performed in time intervals of 14 days or less.

4. The use of any of the preceding claims, wherein said second treatment period is repeated over a period of at least 1 year.

5. The use of any of the preceding claims, wherein 20 units or less are administered per treatment session.

6. The use of any of the preceding claims, wherein said second amount [units] administered during said second treatment period is within 50% to 150% of the first amount administered during the first treatment period.

7. The use of any of the preceding claims, wherein said second amount is administered by an automated device.

8. The use of claim 7, wherein the automated device is a micro pump-device.

9. The use of claim 7 or 8, wherein the administration is continuous.

10. The use of any of the precedings claims, wherein the administration is performed by repeated injection.

11. The use of claim 10, wherein the injection is selected from the group comprising intradermal, subcutaneous, intramuscular and injection.

12. The use of any of the claims 1 to 8, 10 and 11, wherein the administration is intermittent.

13. The use of claim 12, wherein the intermittent administration is a repeated administration in intervals of less than 72 hours.

14. The use of a neurotoxic component of a *Clostridium botulinum* toxin complex, the component being devoid of any other protein component of the *Clostridium botulinum* toxin complex for the removal of facial lines or wrinkles of the skin or facial asymmetries, wherein the component is administered according to the use defined in any of the preceding claims.

Europäisches
Patentamt

European
Patent Office

Office européen
des brevets

## EUROPEAN SEARCH REPORT

**Application Number**

EP 10 00 6608

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2008/000490 A1 (MERZ PHARMA GMBH & CO KGAA [DE]) 3 January 2008 (2008-01-03) <br> * page 5, line 29 - page 6, line 7 * <br> * page 13, line 7 - line 12 * <br> * page 15, line 23 - line 28 * <br> * page 26, line 10 - line 20 * <br> * example 2 * <br> ----- | 1-15 | INV. <br> A61K38/48 <br> A61P43/00 <br> A61P21/02 <br> A61P17/00 <br> A61Q19/08 |
| X | EP 1 985 276 A1 (MERZ PHARMA GMBH & CO KGAA [DE]) 29 October 2008 (2008-10-29) <br> * paragraphs [0012], [0016], [0017], [0070] * <br> ----- | 1-15 | |
| X,D | WO 2010/022979 A1 (MERZ PHARMA GMBH & CO KGAA [DE]; FREVERT JUERGEN [DE]) 4 March 2010 (2010-03-04) <br> * paragraphs [0018], [0033], [0034], [0119] * <br> ----- | 1-15 | |
| | | | TECHNICAL FIELDS SEARCHED (IPC) <br><br> A61K <br> A61P <br> A61Q |

The present search report has been drawn up for all claims

| Place of search | Date of completion of the search | Examiner |
|---|---|---|
| Munich | 23 September 2010 | Pilling, Stephen |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 10 00 6608

This annex lists the patent family members relating to the patent documents cited in the above-mentioned European search report.
The members are as contained in the European Patent Office EDP file on
The European Patent Office is in no way liable for these particulars which are merely given for the purpose of information.

23-09-2010

| Patent document cited in search report | Publication date | Patent family member(s) | Publication date |
|---|---|---|---|
| WO 2008000490 A1 | 03-01-2008 | AR 061669 A1 | 10-09-2008 |
| | | AU 2007264008 A1 | 03-01-2008 |
| | | CA 2654214 A1 | 03-01-2008 |
| | | CL 19112007 A1 | 14-03-2008 |
| | | CN 101460190 A | 17-06-2009 |
| | | EP 2032157 A1 | 11-03-2009 |
| | | JP 2009541396 T | 26-11-2009 |
| | | KR 20090027732 A | 17-03-2009 |
| | | RU 2009102848 A | 10-08-2010 |
| | | US 2008003241 A1 | 03-01-2008 |
| | | UY 30450 A1 | 30-04-2009 |
| | | ZA 200808491 A | 25-11-2009 |
| EP 1985276 A1 | 29-10-2008 | CA 2684529 A1 | 06-11-2008 |
| | | EP 2148641 A1 | 03-02-2010 |
| | | WO 2008131941 A1 | 06-11-2008 |
| | | US 2008279896 A1 | 13-11-2008 |
| WO 2010022979 A1 | 04-03-2010 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008000490 A
- WO 2006027207 A1
- WO 2006114308 A1
- WO 2009015840 A
- WO 2010022979 A
- EP 2009006273 W
- US 5860957 A
- US 6017318 A
- WO 2009023601 A
- DE 102006020402
- US 20070265551 A
- EP 2015065 A

**Non-patent literature cited in the description**

- **BEB GOERTELMEYER R ; BRINKMANN S ; COMES G ; DELCKER A.** The Blepharospasm Disability Index (BSDI) for the Assessment of Functional Health in Focal Dystonia. *Clin. Neurophysiol.,* 2002, vol. 113 (1), S77-S78
- **MUELLER J ; WISSEL J ; KEMMLER G ; BODNER T ; POEWE W.** Quality of life in patients with craniocervical dystonia: development of the CCDQ-24. *Mov. Disord.,* 2000, vol. 15 (3), 76
- **WELMER AK ; VON ARBIN M ; WIDEN HOLMQVIST L ; SOMMERFELD DK.** Spasticity and its association with functioning and health-related quality of life 18 months after stroke. *Cerebrovasc. Dis.,* 2006, vol. 21 (4), 247-253
- **DRESSLER D.** Botulinum Toxin Therapy. Thieme Verlag, 2000
- **SHERIDAN et al.** *J. Appl. Toxicol.,* 1999, vol. 19, S29-S33